(19) Europäisches Patentamt
European Patent Office
Office européen des brevets

(11) Publication number: **0 429 098 A1**

(12) **EUROPEAN PATENT APPLICATION**

(21) Application number: **90124927.6**

(51) Int. Cl.[5]: **G03C 7/38**, //C07D487/04

(22) Date of filing: **08.03.88**

This application was filed on 20 - 12 - 1990 as a divisional application to the application mentioned under INID code 60.

(30) Priority: **09.03.87 US 23517**

(43) Date of publication of application: **29.05.91 Bulletin 91/22**

(60) Publication number of the earlier application in accordance with Art.76 EPC: **0 284 239**

(84) Designated Contracting States: **DE FR GB NL**

(71) Applicant: **EASTMAN KODAK COMPANY**
**Patent Department, 343 State Street**
**Rochester, NY 14650-2201(US)**

(72) Inventor: **Bowne, Arlyce Tolman, c/o EASTMAN KODAK COMPANY**
**Patent Department, 343 State Street**
**Rochester, New York 14650-2201(US)**
Inventor: **Normandin, Sharon Eileen, c/o EASTMAN KODAK COMP.**
**Patent Department, 343 State Street**
**Rochester, New York 14650-2201(US)**
Inventor: **Milner, Nigel Edgewick, c/o EASTMAN KODAK COMPANY**
**Patent Department, 343 State Street**
**Rochester, New York 14650-2201(US)**
Inventor: **Clarke, David, c/o EASTMAN KODAK COMPANY**
**Patent Department, 343 State Street**
**Rochester, New York 14650-2201(US)**

(74) Representative: **Haile, Helen Cynthia et al**
**Kodak Limited Patent Department Headstone Drive**
**Harrow, Middlesex HA1 4TY(GB)**

(54) **Photographic silver halide materials and process comprising a pyrazoloazole coupler.**

(57) A photographic silver halide material contains a novel 1H-pyrazolo(3,2-c)-s-triazole coupler of the formula:

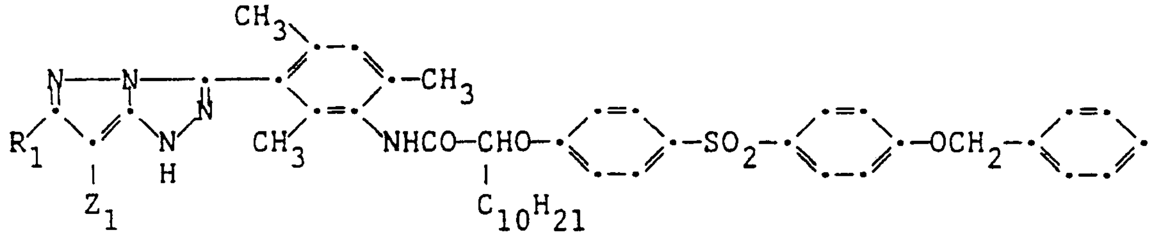

wherein
$R_1$ is hydrogen or a substituent that does not adversely affect the coupler, particularly alkyl containing 1 to 30 carbon atoms, or aryl containing 6 to 20 carbon atoms, or a 5 or 6 member heterocyclic group;
$Z_1$ is hydrogen or a coupling-off group,
forms a magenta dye that has a narrow half-bandwidth (HBW).

EP 0 429 098 A1

# PHOTOGRAPHIC SILVER HALIDE MATERIALS AND PROCESS COMPRISING A PYRAZOLOAZOLE COUPLER

This invention relates to novel pyrazoloazole couplers and to photographic silver halide materials and processes using such couplers enabling formation of a magenta dye that has a desired shift in hue and increased stability.

Color images are customarily obtained by reaction between the oxidation product of a silver halide color developing agent and a dye-forming coupler. Pyrazolone dye-forming couplers are useful for forming magenta dye images; however, pyrazoloazole couplers represent another class of couplers that are useful for this purpose. Examples of such couplers, such as 1H-pyrazolo[3,2-c]-s-triazole couplers, are described in, for example, U.S. Patents 4,443,536 and 4,540,654; U.K. Patents 1,247,493; 1,252,418 and 1,398,979 and European Patent Application Nos. 0 183 444, 0 183 445 and 0 200 206.

While such magenta dye-forming couplers are useful in photographic silver halide materials and processes, many of such couplers provide dyes that do not have the desired properties. Pyrazoloazole couplers, particularly pyrazolotriazole couplers, often form magenta dyes that fall short of desired aims in hue and stability. It has been desirable to provide such dyes that have a narrower absorption half-bandwidth (HBW) to improve hue purity and color saturation.

According to the present invention there is provided a photographic silver halide material comprising a support bearing at least one photographic silver halide emulsion layer and in or adjacent the emulsion layer a dye-forming 1H-pyrazolo(3,2-c)-s-triazole coupler of the formula:

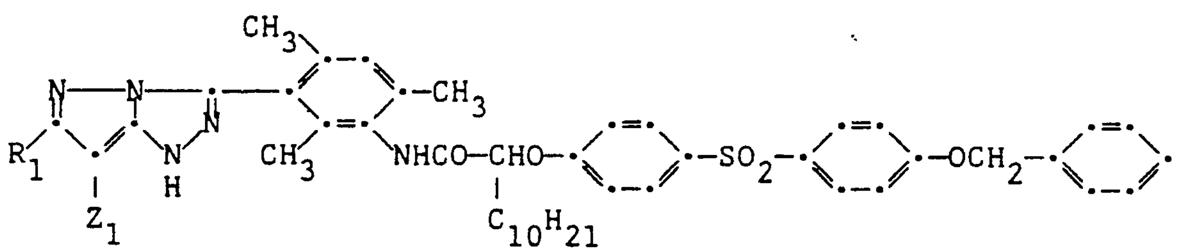

wherein

$R_1$ is hydrogen or a substituent that does not adversely affect the coupler, particularly alkyl containing 1 to 30 carbon atoms, or aryl containing 6 to 20 carbon atoms, or a 5 or 6 member heterocyclic group;

$Z_1$ is hydrogen or a coupling-off group.

The present invention further provides the above couplers *per se*.

Examples of groups which $R_1$ may represent are amino, such as dioctylamino, dimethylamino, and dodecylamino; alkyl, such as alkyl containing 1 to 30 carbon atoms, for example, methyl, ethyl, propyl, n-butyl, t-butyl, octyl and eicosyl; cycloalkyl, such as cyclohexyl and cyclopentyl; aryl, such as aryl containing 6 to 20 carbon atoms, for example, phenyl, naphthyl, and mesityl; carboxy; cyano; nitro; a heterocyclic group, such as a heterocyclic group comprised of atoms selected from carbon, oxygen, nitrogen and sulfur atoms necessary to complete a 5 or 6 member ring, for example, pyrrole, oxazolyl and pyridyl; or $-(L_1)_n-(L_2)_m-R_6$ wherein $L_1$ is a linking group that does not adversely affect the desired properties of the coupler, such as an alkylene, for example, alkylene containing 1 to 20 carbon atoms including methylene, ethylene, propylene, n-butylene, isopropylmethylene, and octylene, or arylene, such as arylene containing 6 to 20 carbon atoms, for example, phenylene and naphthylene; $L_2$ is a linking group that does not adversely affect the desired properties of the coupler, and that is the same as or different from $L_1$, and is typically 0,

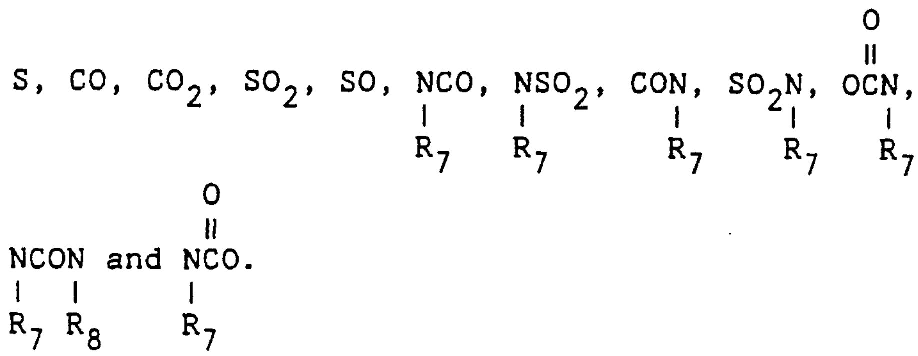

$R_7$ and $R_8$ are individually hydrogen, alkyl, such as alkyl containing 1 to 20 carbon atoms, for example, methyl, ethyl, propyl, n-butyl, t-butyl, and eicosyl, or aryl, such as aryl containing 6 to 20 carbon atoms, for example, phenyl and naphthyl; n and m are individually 0 or 1; and, $R_6$ is alkyl, such as alkyl containing 1 to 30 carbon atoms, for example, methyl, ethyl, propyl, n-butyl, t-butyl, and octyl, or aryl, such as aryl containing 6 to 20 carbon atoms, for example, phenyl, napthyl, and mesityl; or a heterocyclic group, such as a 5- or 6-member heterocyclic group comprised of atoms selected from carbon, nitrogen, oxygen and sulfur atoms necessary to complete a 5- or 6-member heterocyclic ring, such as an oxazole, pyridine, pyrrole or thiophene ring.

These groups are unsubstituted or optionally substituted with groups that do not adversely affect the desired properties of the pyrazoloazole coupler. Examples of useful substituents can include ballast groups and coupler moieties known to be useful in the photographic art, or alkyl, such as alkyl containing 1 to 4 carbon atoms, for example, methyl, ethyl and t-butyl.

The pyrazoloazole contains in the coupling position the group Z, which may be hydrogen or a coupling-off group, also known as a leaving group.

Coupling-off groups, defined by Z herein, are well known to those skilled in the art. Such groups can determine the equivalency of the coupler, can modify the reactivity of the coupler, or can advantageously affect the layer in which the coupler is coated or other layers in the element by performing, after release from the coupler, such functions as development inhibition, development acceleration, bleach inhibition, bleach acceleration, color correction, and the like. Representative classes of coupling-off groups include halogen, particularly chlorine, bromine, or fluorine, alkoxy, aryloxy, heterocyclyloxy, sulfonyloxy, acyloxy, carbonamido, imido, acyl, heterocyclylimido, thiocyano, alkylthio, arylthio, heterocyclylthio, sulfonamido, phosphonyloxy and arylazo. They are described in, for example, U.S. Patents 2,355,169; 3,227,551, 3,432,521; 3,476,563; 3,617,291; 3,880,661; 4,052,212 and 4,134,766; and in U.K. patents and published application numbers 1,466,728; 1,531,927; 1,533,039; 2,006,755A and 2,017,704A.

Examples of specific coupling-off groups are

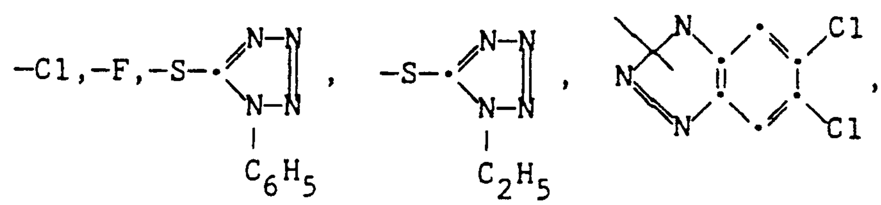

$-SCN$, $-OCH_3$, $-OC_6H_5$, $-OCH_2CONHCH_2CH_2OH$,

$-OCH_2CONHCH_2CH_2OCH_3$, $-OCH_2CONHCH_2CH_2OCOCH_3$,

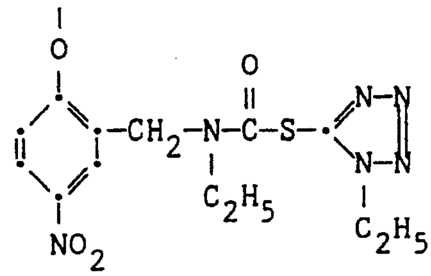

$-NHSO_2CH_3$

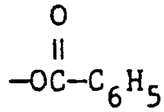

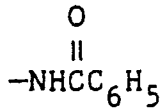

$-OSO_2CH_3$

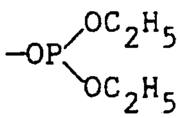

,

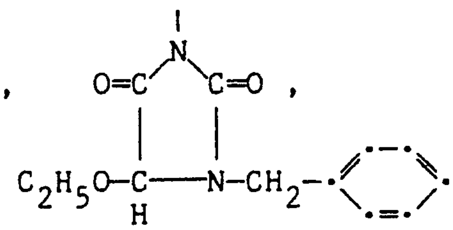

,

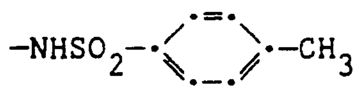

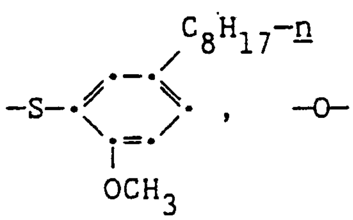

,

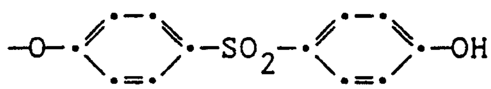

,

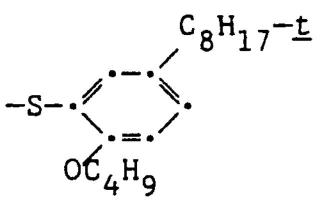

$-S(CH_2)_2COOH$,

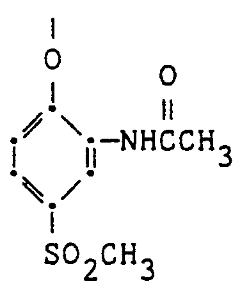

, and

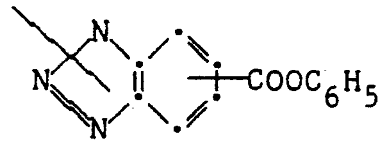

One especially preferred coupler has the formula:

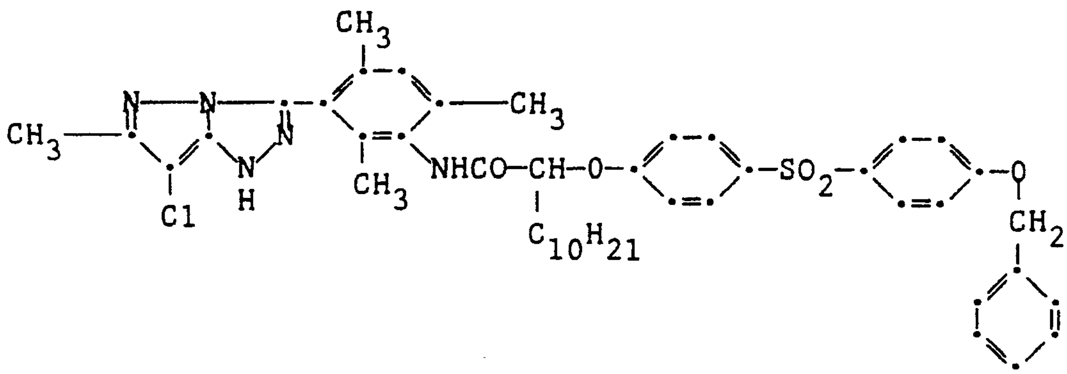

Image dye formed from it has particularly good spectral absorption properties, shows good light fastness and the coupler can be made easily and cheaply.

The pyrazoloazole couplers according to the invention can be used in ways and for purposes that pyrazoloazole couplers have been used in the photographic art.

The pyrazoloazole couplers according to the invention are prepared by the general methods of synthesis described in the art, such as in Research Disclosure, August 1974, Item No. 12443 published by Kenneth Mason Publications, Ltd., The Old Harbourmaster's, 8 North Street, Emsworth, Hampshire P010 7DD, England and U.S. Patent 4,540,654, for example, those methods described in the parent specification (0,284,239).

The photographic materials can be single color elements or multicolor elements. In a multicolor element, the dye-forming coupler of this invention is typically associated with a green-sensitized emulsion, although it could be associated with an unsensitized emulsion or an emulsion sensitized to a different region of the spectrum. Multicolor elements typically contain dye image-forming units sensitive to each of the three primary regions of the spectrum. Each unit can be comprised of a single emulsion layer or of multiple emulsion layers sensitive to a given region of the spectrum. The layers of the element, including the layers of the image-forming units, can be arranged in various orders as known in the art. In an alternative format, the emulsion sensitive to each of the three primary regions of the spectrum can be disposed as a single segmented layer.

A typical multicolor photographic element comprises a support bearing a cyan dye image-forming unit comprised of at least one red-sensitive silver halide emulsion layer having associated therewith at least one cyan dye-forming coupler, a magenta dye image-forming unit comprising at least one green-sensitive silver halide emulsion layer having associated therewith at least one magenta dye-forming coupler of this invention and a yellow dye image-forming unit comprising at least one blue-sensitive silver halide emulsion layer having associated therewith at least one yellow dye-forming coupler. The element can contain additional layers, such as filter layers, interlayers, overcoat layers, subbing layers, and the like.

In the following discussion of examples of materials useful in the emulsions and elements of this invention, reference will be made to Research Disclosure, December 1978, Item No. 17643. This publication will be identified hereafter by the term "Research Disclosure".

The silver halide emulsions employed in the elements of this invention can be either negative-working or positive-working. Examples of useful emulsions and their preparation are described in Research Disclosure Sections I and II and the publications cited therein. Examples of useful vehicles for the emulsion layers of elements of this invention are described in Research Disclosure Section IX and the publications cited therein.

In addition to the couplers of this invention, the elements of the invention can include additional

couplers, such as described in Research Disclosure Section VII, paragraphs D, E, F and G and the publications cited therein. These couplers can be incorporated in the elements and emulsion as described in Research Disclosures of Section VII, paragraph C and the publications cited therein.

The photographic elements of this invention or individual layers thereof, can contain brighteners (see Research Disclosure Section V), antifoggants and stabilizers (See Research Disclosure Section VI), antistain agents and image dye stabilizer (see Research Disclosure Section VII, paragraphs I and J), light absorbing and scattering materials) see Research Disclosure Section VIII), hardeners (see Research Disclosure Section XI), plasticizers and lubricants (see Research Disclosure Section XIII), matting agents (see Research Disclosure Section XVI) bleach accelerator and development modifiers (see Research Disclosure Section XXI) colored masking couplers, and competing couplers.

The photographic elements can be coated on a variety of supports as described in Research Disclosure Section XVII and the references described therein.

Photographic elements can be exposed to actinic radiation, typically in the visible region of the spectrum, to form a latent image as described in Research Disclosure Section XVIII and then processed to form a visible dye image as described in Research Disclosure Section XIX. Processing to form a visible dye image includes the step of contacting the element with a color developing agent to reduce developable silver halide and oxidize the color developing agent. Oxidized color developing agent in turn reacts with the coupler to yield a dye.

Preferred color developing agents are p-phenylene diamines. Especially preferred are
4-amino-3-methyl-N,N-diethylaniline hydrochloride,
4-amino-3-methyl-N-ethyl-N-β-(methanesulfonamido)ethylaniline sulfate hydrate,
4-amino-3-methyl-N-ethyl-N-β-hydroxyethylaniline sulfate,
4-amino-3-β-(methanesulfonamido)ethyl-N,N-diethylaniline hydrochloride and
4-amino-N-ethyl-N-(2-methoxyethyl)-m-toluidine-di-p-toluenesulfonic acid.

With negative working silver halide this processing step leads to a negative image. To obtain a positive (or reversal) image, this step can be preceded by development with a non-chromogenic developing agent to develop exposed silver halide, but not form dye, and then uniform fogging of the element to render unexposed silver halide developable. Alternatively, a direct positive emulsion can be employed to obtain a positive image.

Development is followed by the conventional steps of bleaching, fixing, or bleach-fixing, to remove silver and silver halide, washing and drying.

## Claims

1. A photographic silver halide material comprising a support bearing at least one photographic silver halide emulsion layer and in or adjacent the emulsion layer a dye-forming 1H-pyrazolo(3,2-c)-s-triazole coupler of the formula:

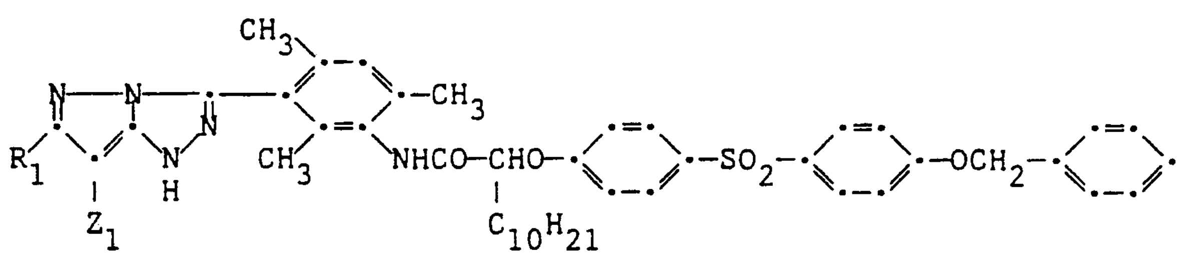

wherein
$R_1$ is hydrogen or a substituent that does not adversely affect the coupler;
$Z_1$ is hydrogen or a coupling-off group.

2. A photographic silver halide material as claimed in claim 1 wherein $R_1$ is an alkyl containing 1 to 30 carbon atoms; an aryl containing 6 to 20 carbon atoms, or a 5 or 6 member heterocyclic group.

3. A photographic silver halide material as claimed in claim 1 or 2 wherein the 1H-pyrazolo[3,2-c]- s-triazole coupler has the formula:

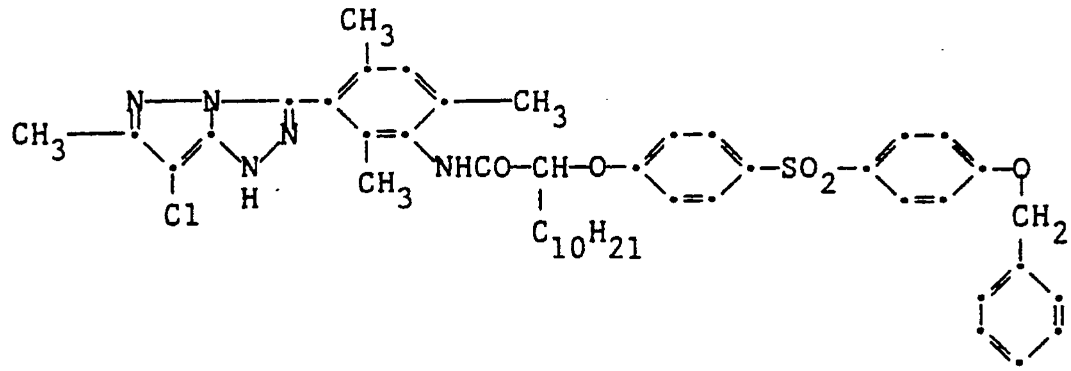

4. A multicolor photographic silver halide material as claimed in any of claims 1-3 comprising a red-sensitive silver halide emulsion unit having associated therewith a cyan dye image providing material, a green-sensitive silver halide emulsion unit having associated therewith a magenta dye image-providing material, and a blue-sensitive silver halide emulsion unit having associated therewith a yellow dye image-providing silver halide material wherein the pyrazoloazole coupler is in at least one of said units.

5. A process of forming a magenta dye image in an exposed photographic material as defined in any of claims 1-4, said process comprising developing the exposed photographic material with a silver halide color developing agent.

6. A dye-forming pyrazoloazole coupler as defined in any of claims 1-3.

European Patent Office

# EUROPEAN SEARCH REPORT

Application Number

EP 90 12 4927

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4 ) |
|---|---|---|---|
| | no further documents have been disclosed<br>----- | | G03C7/38<br>C07D487/04 |
| | | | **TECHNICAL FIELDS SEARCHED (Int. Cl.4 )** |
| | | | G03C |

The present search report has been drawn up for all claims

| Place of search | Date of completion of the search | Examiner |
|---|---|---|
| THE HAGUE | 19 MARCH 1991 | PHILOSOPH L. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..............................................
& : member of the same patent family, corresponding document



EPO FORM 1503 03.82 (P0401)